# FASCICULE DE BREVET EUROPEEN

(11) **EP 0 714 709 B1**
(45) Date de publication et mention de la délivrance du brevet: **14.06.2000**
(21) Numéro de dépôt: 95402679.5
(22) Date de dépôt: 29.11.1995
(51) Int. Cl.: B05B 12/00, B05B 17/06

(54) **Disposif d'émission de liquides**
Vorrichtung zum Ausströmen einer Flüssigkeit
Device for emitting a liquid

(30) Priorité: 29.11.1994 FR 9414301
(43) Date de publication de la demande: 05.06.1996
(73) Titulaire: Millet, Jean-Claude, 26500 Bourg les Valence (FR); Millet, Cyril, 26500 Bourg les Valence (FR)
(72) Inventeur: Millet, Jean-Claude, 26500 Bourg les Valence (FR); Millet, Cyril, 26500 Bourg les Valence (FR)
(74) Mandataire: Ropital-Bonvarlet, Claude

(56) Documents cités:
- WO-A-94/21317
- WO-A-94/22592
- DE-A- 3 434 334
- DE-A- 3 919 447
- DE-U- 8 632 302
- US-A- 4 521 786
- US-A- 4 702 418

## Description

L'invention concerne un dispositif d'émission de liquides, se présentant notamment sous forme de parfums, d'arômes ou de liquides à principe actif ou non, permettant par exemple des assemblages d'odeurs programmés à des fins de type ludique, de plaisir, de confort, de traitement biologique, d'alarme ou bien en rapport avec la santé par la reconstruction d'environnements dépendants du cycle circadien.

Actuellement, il existe des dispositifs de pulvérisation de parfum à usage personnel ou collectif se présentant soit sous forme de bombe aérosol dans laquelle la pulvérisation en gouttelettes est réalisée grâce à l'énergie d'un gaz sous pression, soit sous forme d'un vaporisateur dans lequel la pulvérisation est obtenue grâce à l'énergie mécanique provenant de l'actionnement d'un système mécanique par l'utilisateur, ou encore à partir d'un système à ultrasons.

Ces dispositifs actuels présentent principalement deux sortes d'inconvénients : d'une part, la pulvérisation se fait en brouillard à partir d'un volume de liquide mal contrôlé et d'autre part, ils ne diffusent à chaque utilisation que le parfum résultant d'une préparation élaborée avant son stockage dans les dispositifs.

Il existe également un dispositif d'émission de liquides, décrit dans la demande de brevet internationale, publiée sous le numéro WO 94/22592, utilisant un procédé de gouttes à la demande et doté d'un circuit électronique de commande alimenté par une batterie. Le document WO 94/22592 forme la base du préambule de la revendication 1.

Pour résoudre ces inconvénients, le but de la présente invention est de réaliser un dispositif d'émission de liquides présentant les deux propriétés suivantes :
- il projette un nombre déterminé de gouttes calibrées dès sa sollicitation,
- il possède plusieurs réservoirs de liquides différents, se présentant sous forme de cartouches amovibles constituant une première partie d'un boîtier rendue solidaire d'une seconde partie du boîtier supportant des moyens de projection, les moyens de commande associés à des moyens de sécurité, des moyens d'activation et des moyens d'alimentation électrique. Les liquides sont notamment des parfums, alimentant simultanément ou séquentiellement des moyens de projection de gouttes de telle sorte que leur combinaison à l'extérieur du dispositif conduise à un assemblage particulier, par exemple à la création d'un nouveau parfum.

Ce dispositif se présente sous deux formes, une forme portative en vue d'une utilisation individuelle sur la personne humaine et une forme plus encombrante de matériel de bureau, ou d'appartement, autorisant éventuellement un usage collectif dans l'atmosphère et une plus grande sophistication de chacune des fonctions élémentaires.

Pour cela, l'objet de l'invention est un dispositif d'émission de liquides dans un boîtier, comportant :
- au moins un réservoir de stockage de liquide,
- des moyens de projection de gouttes calibrées du type goutte à la demande,
- des moyens de commande programmables desdits moyens de projection, contrôlant l'émission d'un seul liquide ou de plusieurs liquides simultanément ou séquentiellement, associés à des moyens de sécurité contrôlant le passage du dispositif d'émission d'un état de sommeil à un état de veille pour éviter toute projection intempestive,
- des moyens d'activation des moyens de commande,
- des moyens d'alimentation électrique,
caractérisé en ce qu'il comporte au moins deux réservoirs de stockage qui sont des cartouches amovibles constituant une première partie du boîtier rendue solidaire d'une seconde partie du boîtier supportant les moyens de projection, les moyens de commande, les moyens d'activation, les moyens d'alimentation électrique, ainsi que les moyens de sécurité.

D'autres caractéristiques et avantages de l'invention apparaîtront à la lecture de la description suivante d'un exemple particulier non limitatif de dispositif d'émission de liquides, cette description étant faite en relation avec les dessins joints dans lesquels :
- la figure 1 est un schéma des éléments constitutifs d'un dispositif selon l'invention ;
- les figures 2ₐ et 2_{b} sont respectivement des vues en perspective et en éclatée d'un premier mode de réalisation d'un dispositif selon l'invention, dans une version portative ;
- la figure 3 est un schéma du premier mode de réalisation d'un dispositif selon l'invention;
- la figure 4 est une vue en perspective d'un second mode de réalisation d'un diffuseur de parfum selon l'invention ;
- les figures 5ₐ à 5ₑ sont des vues en coupe d'un schéma du second mode de réalisation d'un diffuseur de parfum selon l'invention.

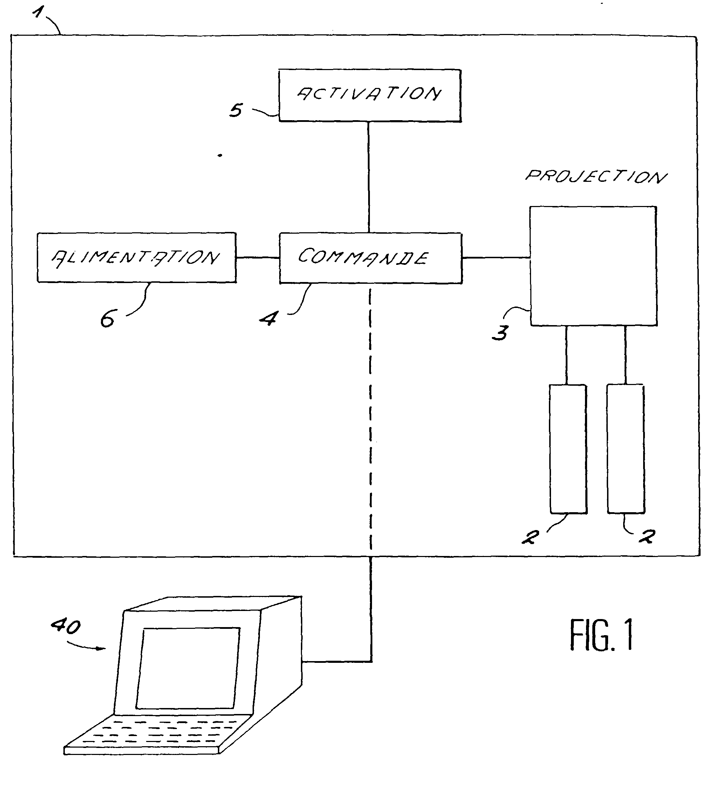

Un dispositif d'émission de liquides, en particulier de parfums, selon l'invention a pour caractéristique essentielle d'émettre un liquide ou plusieurs liquides simultanément ou séquentiellement, de telle manière que leur combinaison à l'extérieur du dispositif, notamment leur évaporation dans l'air, conduise à un assemblage particulier. A cet effet, comme le montre le schéma de la figure 1, il comporte un boîtier 1 duquel plusieurs réservoirs 2 de liquides constituent une partie. Selon un exemple de réalisation de l'invention, chaque réservoir contient un parfum ou une substance aromatique liquide, pure ou diluée ou bien encore sous forme plus complexe comme des encapsulations, des microémulsions ou des mélanges.

Chaque réservoir 2 de liquide est relié à des moyens 3 de projection de gouttes calibrées selon le principe de goutte à la demande. Ces moyens sont, par exemple, des têtes de projection du type thermique - ou bubble jet - comprenant plusieurs canaux d'éjection dans chacun desquels sont créées par chauffage des bulles provoquant l'apparition des gouttes.

Selon un autre exemple de réalisation, les moyens de projection de gouttes calibrées selon le principe de goutte à la demande sont des têtes de projection du type piézo-électrique, comprenant plusieurs canaux d'éjection soumis à des vibrations créées par des moyens piézo-électriques engendrant la formation de gouttes.

Selon une caractéristique de l'invention, ces têtes de projection de gouttes comportent un nombre important de canaux d'éjection qui sont totalement intégrés dans une matrice de silicium et permettent l'émission de 128 jets de parfum par exemple.

Ces moyens de projection ont l'avantage de générer des gouttes calibrées de petite dimension compatible avec la projection d'un liquide tel qu'un parfum, tout en étant économiques, compacts et nécessitant peu d'énergie.

Dans le boîtier 1 sont également disposés des moyens de commande 4 programmables des têtes de projection de gouttes, destinés à contrôler l'émission d'un seul liquide, ou de plusieurs liquides simultanément ou séquentiellement, à chaque utilisation. Les programmes qui commandent les moyens de projection sont mis en mémoire dans le dispositif et dépendent de l'application envisagée. Ils peuvent aussi être apportés de manière interactive par une source extérieure, tel qu'un ordinateur 40.

Des moyens d'activation 5 desdits moyens de commande sont constitués par un bouton à piloter par pression ou par effleurement avec le doigt, ou par un détecteur de proximité, ou par un système d'horloge programmable. Dans le cas de l'utilisation d'un détecteur de proximité, les moyens de commande comprennent également une temporisation destinée à limiter le nombre d'émissions à chaque approche du dispositif d'une surface déclenchant la projection.

Enfin, des moyens d'alimentation électrique 6 sont prévus et peuvent comporter des batteries, de manière à rendre le dispositif autonome.

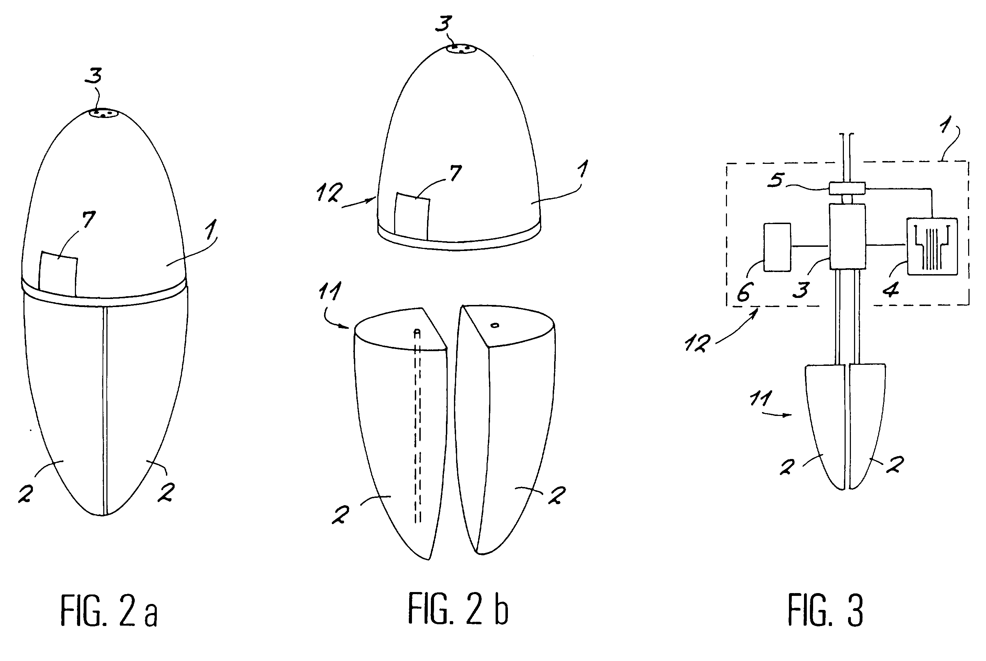

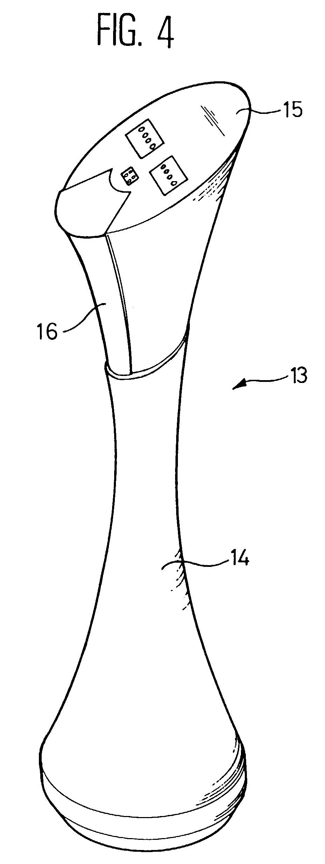

Dans le premier mode de réalisation du dispositif d'émission, dans sa version portative, sur les figures 2ₐ et 2_{b} représentant respectivement une vue en perspective et une vue en éclaté et sur la figure 4 qui en représente un schéma, les réservoirs 2 de parfums constituent une première partie 11 du boîtier 1 et viennent s'emboîter dans une seconde partie 12 à laquelle elle est rendue solidaire par une attache ou un clip, et dans laquelle sont logés les autres moyens du dispositif. Dans ce mode de réalisation, la seconde partie 12 du boîtier est dotée d'une trappe d'accès pour le remplacement des moyens d'alimentation électrique, telle une pile par exemple du type pile-bouton.

Les moyens de commande sont gérés par un logiciel personnalisé.

Les moyens d'activation de ces moyens de commande des têtes de projection de parfums sont constitués soit par un bouton soit par un détecteur de proximité 5, disposés par rapport à la zone de sortie des buses d'éjection des moyens de projection 3, de telle manière que l'émission de parfums puisse être tournée vers la surface à parfumer tandis que ce bouton ou ce détecteur est activé. Dans le cas d'un détecteur de proximité, qui peut être du type infrarouge, capacitif ou acoustique intégré dans du silicium, celui-ci se situe dans une zone très proche des organes de projection. Lorsqu'on approche le dispositif selon l'invention de la surface destinée à recevoir la projection des parfums, le détecteur de proximité active les buses d'éjection et la temporisation limite le nombre d'émissions de gouttes de parfums déclenchées par chaque approche du dispositif.

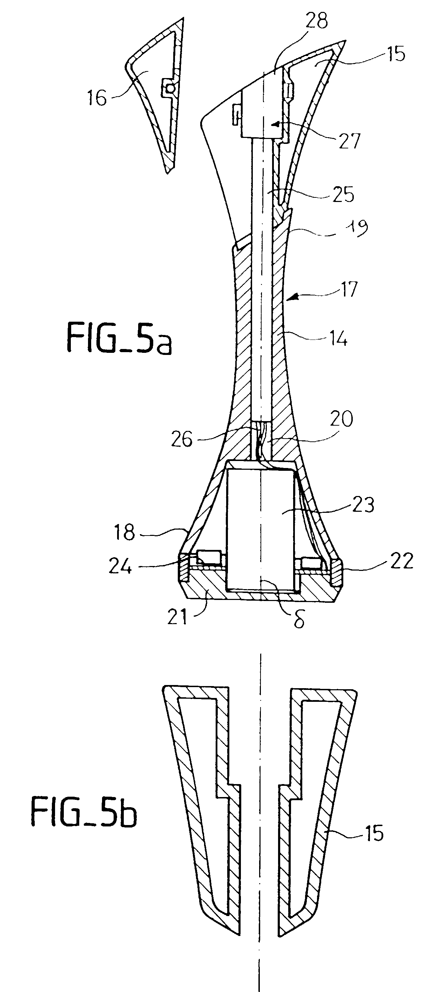

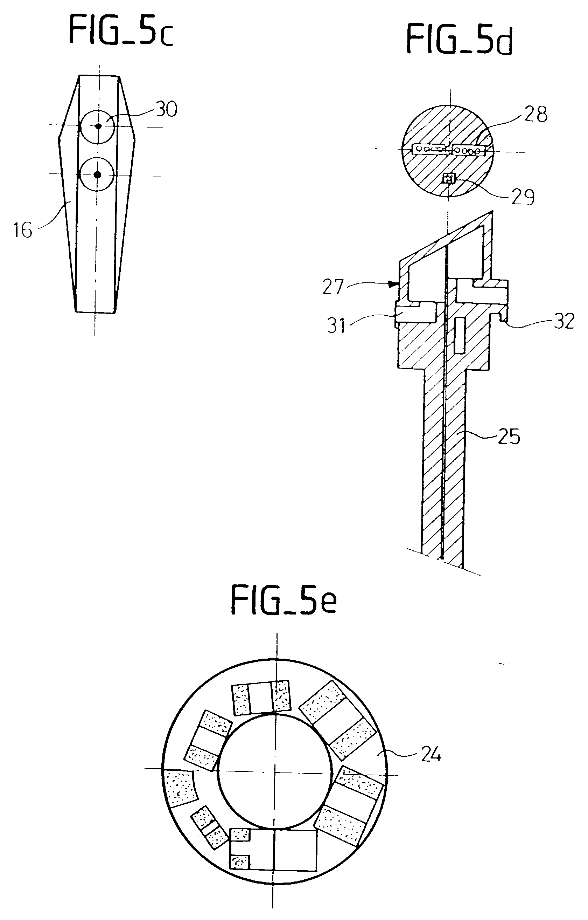

Selon un second mode de réalisation d'un dispositif selon l'invention, réalisant un diffuseur de parfum comme le montre les figures 4 à 5e, celui-ci se présente sous la forme d'un boîtier 13 constitué d'un flacon 14 sur lequel s'adaptent au moins deux cartouches 15 et 16 de parfum, extérieures au flacon 14. De façon non limitative, le flacon est de forme allongée et de section droite circulaire dont le rayon est variable le long de l'axe longitudinal δ de façon à présenter une partie centrale 17 plus étroite que ses deux extrémités 18 et 19. Selon la vue en coupe longitudinale de la figure 5ₐ, le flacon 14 est creusé par une cavité cylindrique 20 tout au long de l'axe longitudinal δ. Dans le cas d'un flacon évasé, le diamètre de cette cavité augmente vers l'extrémité inférieure 18 qui sert d'embase au flacon. Un bouchon 21 servant d'embase au flacon est prévu sous ladite extrémité inférieure 18 pour obturer le flacon, auquel il est fixé par une bague 22.

A l'intérieur de l'extrémité inférieure de la cavité 20 sont logés les moyens d'alimentation électrique constitués par une carte électronique 24 de puissance, en forme d'anneau (figure 5ₑ), et une pile électrique 23 de type cylindrique par exemple, disposée au centre de l'anneau et maintenue par le bouchon 21. Dans la cavité cylindrique 20 creusant le flacon 14, on glisse un tube 25 cylindrique rigide, servant d'une part de support aux têtes de projection de parfums 28 et à un détecteur de proximité 29 rendus ainsi solidaires du flacon 14 (figure 5_{d}), et servant d'autre part au passage de conducteurs électriques 26 reliés à la carte électronique 24 de puissance. Dans le cas particulier décrit sur la figure 5ₐ, le tube 25 a une longueur supérieure à celle de la partie de la cavité 20 correspondant à la partie centrale 17 du flacon 14, et un diamètre extérieur sensiblement égal au diamètre de la cavité, excepté à son extrémité supérieure 27 où le diamètre est supérieur pour supporter les têtes de projection. Chaque tête de projection 28 est alimentée en parfum par une cartouche amovible dont l'ouverture 30 communique avec une canalisation d'entrée 31 autorisant la circulation du parfum vers la tête.

Selon l'exemple représenté, les deux cartouches sont de tailles différentes. La cartouche dite principale 15 contient un premier type de parfum que l'on personnalise par le contenu de la cartouche dite secondaire 16. Dans l'exemple de la figure 5_{b}, la cartouche principale 15 a une forme conique, évasée vers le haut, la cartouche secondaire représentée sur la figure 5_{c} ayant la forme d'un secteur de cône venant s'encastrer dans la cartouche principale. Ces deux cartouches sont rendues solidaires du flacon 14 pour former un diffuseur de parfum, par emboîtement par exemple dans un petit crochet 32 prévu à cet effet à l'extérieur du tube 25 de support des têtes de projection 28. Les cartouches de parfums peuvent être faites en matière plastique, transparente ou non, et sont remplaçables aisément soit lorsqu'elles sont vides soit lorsque l'on veut changer de parfums. Ainsi, l'intérêt principal de cette invention réside dans la possibilité de créer une fragrance personnelle à partir d'au moins deux parfums qui se mélangent à l'extérieur du flacon. Ce diffuseur de parfum a l'avantage de générer, sous forme de gouttes calibrées, un volume parfaitement dosé de chaque parfum composant le mélange.

Les moyens électroniques 4 de commande des têtes de projection et du détecteur de proximité peuvent être gérés par un logiciel personnalisé installé dans des moyens intégrés ou extérieurs au dispositif d'émission.

La structure du diffuseur de parfum qui vient d'être décrit est telle que la pile et les différents éléments assemblés coopèrent afin que le centre de gravité soit situé dans les deux tiers inférieurs de la hauteur du diffuseur pour des raisons de stabilité.

Pour assurer la sécurité du dispositif en empêchant toute émission intempestive de liquide, le déclenchement de la projection se fait en deux étapes à partir d'un état de sommeil dans lequel se trouve généralement le diffuseur, quelque soit l'état de son environnement. Par contre, dès qu'il est pris en main, il passe en état de veille et la projection est déclenchée à partir de cet état quand le détecteur de proximité est suffisamment près de la surface à parfumer. Pour cela, des moyens de sécurité sont prévus pour contrôler le passage de cet état de sommeil à un état de veille et sont constitués par un système d'électrodes associé à un circuit électrique et disposé sur le flacon de telle manière que, lorsque ce dernier est pris en main, la main de l'utilisateur ferme ledit circuit électrique. Les moyens 4 électroniques de commande du diffuseur, recevant un signal électrique, interprètent cette fermeture en le faisant passer de l'état de sommeil à l'état de veille, avant de commander le déclenchement d'une projection d'un volume dosé de gouttes calibrées de parfums.

Dans un exemple particulier de réalisation, les électrodes peuvent être constituées par quatre bandes conductrices transparentes disposées à 90° les unes des autres à la surface du flacon et reliées deux à deux aux moyens électroniques 4 de commande du diffuseur de parfum.

Réalisé sous forme de matériel de bureau, le dispositif émetteur de liquides selon l'invention peut être doté d'un logiciel de commande interactif résidant dans un ordinateur 40 personnel multimédia auquel le dispositif est relié et dont l'interactivité résulte d'éléments de visualisation, tel qu'un afficheur, d'éléments de personnalisation destinés à distinguer le type d'application ou les besoins de son usager dans une application particulière, et d'éléments d'informations sonores, tels qu'une sonnerie, ces derniers étant destinés soit à donner un résultat, soit à avertir de la mise en service d'une fonction, soit à être en rapport avec des fonctions annexes classiques pour un équipement disposant d'une base de temps. Selon un mode de réalisation simple conforme à l'invention, la personnalisation se fait par un clavier à partir d'un mode menu, et selon un autre mode de réalisation plus élaboré, elle se fait par la connexion soit à un support d'informations tel une carte à puce, soit à un logiciel d'ordinateur.

De façon générale, l'interface entre le dispositif selon l'invention et l'organisme vivant susceptible de sentir les émanations volatiles des parfums émis est l'air, mais ce peut être aussi un support intermédiaire tel que du papier ou tout support physique, fibreux ou non, destiné à emmagasiner des informations olfactives pour prolonger leur effet ou la portée spatiale de cet effet, sans sortir du cadre de l'invention.

Comme cela a été mentionné au début de la description, un tel dispositif concerne le domaine de la régulation de type circadien relative aux odeurs produites par la nature aux différentes saisons, et le domaine de la régulation de type génétique relative notamment aux phéromones à l'origine de comportements spécifiques des êtres vivants. Une autre application concerne la régulation de type alarme, permettant la détection de certaines maladies, comme la maladie d'Alzheimer, se caractérisant par la perte de perception d'un certain nombre d'odeurs. Dans ces applications, le dispositif se présente sous forme d'un équipement de bureau de petite taille, autonome ou connecté au dispositif multimédia de son utilisateur. Le nombre de cartouches d'odeurs fondamentales, disponibles et connectables au dispositif, permet de reconstituer une très large variété d'odeurs par la composition de mélanges et leur rythme de diffusion.

Un exemple de dispositif d'émission de parfums portatif réalisable à ce jour selon le premier mode de réalisation décrit, comporte deux cartouches de parfums de 14 ml et 2 ml de volumes respectifs et une pile de 3 Volts et 1300 mAh, utilisant deux têtes de 16 buses chacune. A chaque utilisation, 30 000 à 50 000 gouttes d'environ 5.10⁻⁴ mm³ sont émises pendant une durée d'émission inférieure à 1 seconde. La consommation énergétique de l'ensemble offre une autonomie supérieure à un an et demi, pour cinq utilisations journalières.

## Revendications

1. Dispositif d'émission de liquides dans un boîtier (1), comportant :
- au moins un réservoir (2) de stockage de liquide,
- des moyens de projection (3) de gouttes calibrées du type goutte à la demande,
- des moyens de commande (4) programmables desdits moyens de projection, contrôlant l'émission d'un seul liquide ou de plusieurs liquides simultanément ou séquentiellement, et associés à des moyens de sécurité contrôlant le passage du dispositif d'émission d'un état de sommeil à un état de veille pour éviter toute projection intempestive,
- des moyens d'activation (5) des moyens de commande,
- des moyens d'alimentation électrique (6),
caractérisé en ce qu'il comporte au moins deux réservoirs de stockage (2) qui sont des cartouches amovibles constituant une première partie du boîtier rendue solidaire d'une seconde partie du boîtier supportant les moyens de projection (3), les moyens de commande (4), les moyens d'activation (5), les moyens d'alimentation électrique (6), ainsi que les moyens de sécurité.

2. Dispositif d'émission selon la revendication 1, caractérisé en ce que la seconde partie du boîtier (13) est un flacon (14), creusé selon son axe longitudinal (6) par une cavité cylindrique (20) et obturé à son extrémité inférieure (18) par un bouchon (21) lui servant d'embase et auquel il est fixé par une bague (22), et à l'intérieur duquel est introduit un tube (25) cylindrique rigide servant de support aux moyens de projection (28) à l'extrémité supérieure (19) du flacon et de guidage de conducteurs électriques (26) reliant lesdits moyens de projection aux moyens (6) d'alimentation électrique, et en ce que les cartouches de parfum sont placées à l'extérieur du flacon et rendues solidaires de son extrémité supérieure (19).

3. Dispositif d'émission selon la revendication 2, caractérisé en ce que chaque cartouche de parfum (15, 16) comporte une ouverture (30) de communication avec une canalisation d'entrée (31) du tube (25) autorisant la circulation du parfum vers les moyens de projection (28), et est rendue solidaire du flacon (14) par emboîtement dans un crochet (32) prévu à l'extérieur du tube (25).

4. Dispositif d'émission selon la revendication 1, caractérisé en ce qu'il comporte des moyens de sécurité constitués par un système d'électrodes associé à un circuit électrique, disposé sur le boîtier de telle manière que, lorsque ce dernier est pris en main, la main de l'utilisateur ferme ledit circuit électrique qui envoie un signal aux moyens (4) électroniques de commande du dispositif pour le faire passer d'un état de sommeil à un état de veille.

5. Dispositif d'émission selon la revendication 2, caractérisé en ce que les moyens d'alimentation (6) sont constitués par une carte électronique de puissance (24) associée à une pile électrique, placées dans la partie inférieure du flacon (14) et accessibles par le bouchon (21) de fermeture du flacon (14).

6. Dispositif d'émission selon les revendications 1 et 2, caractérisé en ce que les moyens de commande (4) sont gérés par un logiciel personnalisé installé dans des moyens intégrés ou extérieurs au dispositif.

7. Dispositif d'émission selon la revendication 1, caractérisé en ce que les moyens de commande (4) sont gérés par un logiciel interactif résidant dans un ordinateur personnel multimédia auquel le dispositif est relié.

8. Dispositif d'émission selon la revendication 1, caractérisé en ce que les moyens de commande (4) comprennent une temporisation destinée à limiter le nombre d'émissions à chaque approche du dispositif d'une surface déclenchant la projection.

9. Dispositif d'émission selon la revendication 1, caractérisé en ce que les moyens de projection (3) de gouttes calibrées selon le principe de goutte à la demande comprennent plusieurs canaux d'éjection, totalement intégrés dans une matrice de silicium et sont soit du type thermique, avec création par chauffage, dans chaque canal, de bulles provoquant l'apparition des gouttes, soit du type piézoélectrique, pour lesquels les canaux d'éjection sont soumis à des vibrations créées par des moyens piézo-électriques engendrant la formation de gouttes.

10. Dispositif d'émission selon les revendications 1, 2, 6, 7 et 8, caractérisé en ce que les moyens (5) d'activation desdits moyens (4) de commande sont constitués par un détecteur de proximité, situé au voisinage de la zone de sortie des moyens (3) de projection, activant lesdits moyens (3) de projection dont le nombre d'émissions de gouttes, déclenchées par chaque approche du dispositif d'une surface à parfumer, est limité par la temporisation.

11. Utilisation du dispositif d'émission selon la revendication 1, caractérisé en ce que l'activation des moyens de commande (4) s'accompagne de l'émission d'informations visuelles ou sonores.

12. Utilisation du dispositif d'émission selon la revendication 1, caractérisé en ce que les moyens (4) de commande programmables commandent la projection cyclique d'une composition de parfums conforme à l'obtention d'une odeur dont la non détection est le signe précurseur d'une maladie.

13. Utilisation du dispositif d'émission selon la revendication 1, caractérisé en ce que les moyens (4) de commande programmables commandent la projection d'une composition de parfums conforme à une caractéristique prédéterminée telle qu'une saison ou une position astrale de l'utilisateur.

## Patentansprüche

1. Vorrichtung zum Abgeben von Flüssigkeiten in einem Gehäuse (1), mit
- wenigstens einem Behälter (2) zur Flüssigkeitsspeicherung,
- Spritzeinrichtungen (3) für abgemessene Tropfen nach Anforderung,
- programmierbare Einrichtungen (4) zur Steuerung der genannten Spritzeinrichtungen, welche die Abgabe einer einzigen Flüssigkeit oder mehrerer Flüssigkeiten gleichzeitig oder nacheinander steuern und mit Sicherheitseinrichtungen verbunden sind, die das Umschalten der Abgabevorrichtung aus einem Ruhe- in einen Bereitschaftszustand steuern, um jedes unabsichtliche Ausspritzen zu vermeiden,
- Einrichtungen (5) zum Einschalten der Steuereinrichtungen,
- Stromversorgungseinrichtungen (6),
dadurch gekennzeichnet, daß sie wenigstens zwei Speicherbehälter (2) aufweist, die abnehmbare Patronen sind, welche einen ersten Abschnitt des Gehäuses bilden, der mit einem zweiten Abschnitt des Gehäuses fest verbunden ist, der die Spritzeinrichtungen (3), die Steuereinrichtungen (4), die Einschalteinrichtungen (5), die Stromversorgungseinrichtungen (6) sowie die Sicherheitseinrichtungen trägt.

2. Abgabevorrichtung nach Anspruch 1,
dadurch gekennzeichnet, daß der zweite Abschnitt des Gehäuses (13) ein Flakon (14) ist, der durch eine zylindrische Ausnehmung (20) entlang seiner Längsachse (δ) hohl und an seinem unteren Ende (18) durch einen Stopfen (21) verschlossen ist, welcher ihm als Standfläche dient und an dem er mit einem Ring (22) befestigt ist, und in dessen Innern ein starres zylindrisches Rohr (25) eingeschoben ist, das als Träger der Spritzeinrichtungen (28) am oberen Ende (19) des Flakons und als Führung für elektrische Leiter (26) dient, welche die genannten Spritzeinrichtungen mit den Stromversorgungseinrichtungen (6) verbinden, und dadurch, daß die Parfümpatronen an der Außenseite des Flakons angeordnet und mit dessen oberem Ende (19) fest verbunden sind.

3. Abgabevorrichtung nach Anspruch 2,
dadurch gekennzeichnet, daß jede Parfümpatrone (15, 16) eine Öffnung (30) zur Verbindung mit einem Einlaßkanal (31) des Rohres (25) aufweist, der das Strömen des Parfüms zu den Spritzeinrichtungen (28) ermöglicht, und mit dem Flakon (14) durch Einstecken in einen an der Außenseite des Rohres (25) vorgesehenen Haken (32) fest verbunden ist.

4. Abgabevorrichtung nach Anspruch 1,
dadurch gekennzeichnet, daß sie Sicherheitseinrichtungen aufweist, die von einem Elektrodensystem gebildet sind, das einem elektrischen Kreis verbunden ist, der am Gehäuse in der Weise angeordnet ist, daß, wenn letzteres in die Hand genommen wird, die Hand des Benutzers den genannten elektrischen Kreis schließt, der den elektronischen Steuereinrichtungen (4) der Vorrichtung ein Signal sendet, um diese aus einem Ruhe- in einen Bereitschaftszustand zu schalten.

5. Abgabevorrichtung nach Anspruch 2,
dadurch gekennzeichnet, daß die Versorgungseinrichtungen (6) von einer elektronischen Leistungskarte (24) gebildet sind, die mit einer elektrischen Batterie verbunden ist, welche im unteren Abschnitt des Flakons (14) angeordnet und über den Verschlußstopfen (21) des Flakons (14) erreichbar sind.

6. Abgabevorrichtung nach den Ansprüchen 1 und 2,
dadurch gekennzeichnet, daß die Steuereinrichtungen (4) von einem anwendungsspezifischen Programmpaket verwaltet werden, das in Einrichtungen installiert ist, die in die Vorrichtung integriert oder außerhalb derselben angeordnet sind.

7. Abgabevorrichtung nach Anspruch 1,
dadurch gekennzeichnet, daß die Steuereinrichtungen (4) von einem interaktiven Programmpaket verwaltet werden, das in einem Multimedia-Personalrechner gespeichert ist, mit dem die Vorrichtung verbunden ist.

8. Abgabevorrichtung nach Anspruch 1,
dadurch gekennzeichnet, daß die Steuereinrichtungen (4) eine Verzögerungsschaltung umfassen, die dazu bestimmt ist, die Anzahl der Abgaben bei jeder Annäherung der Vorrichtung an eine das Ausspritzen auslösende Fläche zu begrenzen.

9. Abgabevorrichtung nach Anspruch 1,
dadurch gekennzeichnet, daß die Spritzeinrichtungen (3) für abgemessene Tropfen nach Anforderung mehrere Ausspritzkanäle umfassen, die vollständig in eine Silizium-Matrix integriert und vom thermischen Typ, bei dem durch Erwärmen in jedem Kanal Blasen erzeugt werden, die das Entstehen von Tropfen hervorrufen, oder vom piezoelektrischen Typ sind, bei dem die Ausspritzkanäle durch piezoelektrische Einrichtungen erzeugten Schwingungen ausgesetzt werden, die Tropfen entstehen lassen.

10. Abgabevorrichtung nach den Ansprüchen 1, 2, 6, 7 und 8,
dadurch gekennzeichnet, daß die Einrichtungen (5) zum Einschalten der genannten Steuereinrichtungen (4) von einem Näherungsschalter gebildet sind, der in der Nähe des Auslaßbereiches der Spritzeinrichtungen (3) angeordnet ist und die Spritzeinrichtungen (3) einschaltet, deren Zahl der Abgaben von Tropfen, die bei jeder Annäherung der Vorrichtung an eine zu parfümierende Fläche ausgelöst werden, durch die Verzögerungsschaltung begrenzt wird.

11. Verwendung der Abgabevorrichtung nach Anspruch 1,
dadurch gekennzeichnet, daß das Einschalten der Steuereinrichtungen (4) von der Abgabe von sicht- oder hörbaren Informationen begleitet ist.

12. Verwendung der Abgabevorrichtung nach Anspruch 1,
dadurch gekennzeichnet, daß die programmierbaren Steuereinrichtungen (4) das zyklische Ausspritzen einer Parfümkomposition steuert, die der Entstehung eines Geruchs entspricht, dessen Nichtwahrnehmung das Vorzeichen einer Krankheit ist.

13. Verwendung der Abgabevorrichtung nach Anspruch 1,
dadurch gekennzeichnet, daß die programmierbaren Steuereinrichtungen (4) die Ausspritzen einer Parfümkomposition steuern, die einem vorbestimmten Merkmal, z.B. einer Jahreszeit oder einer Gestirnstellung des Benutzers entspricht.

## Claims

1. A liquid emission device in a housing (1), the device comprising:
· at least one liquid storage tank (2);
· spray means (3) for spraying calibrated drops of the drops-on-demand type;
· programmable control means (4) for controlling said spray means, to control the emission of a single liquid or of a plurality of liquids simultaneously or sequentially, and associated with safety means for causing the device to switch from a sleep state to a standby state to avoid any untimely spraying;
· activation means (5) for activating the control means; and
· electrical power supply means (6);
the device being characterized in that it includes at least two storage tanks (2) which are removable cartridges constituting a first portion of the housing and secured to a second portion of the housing which supports the spray means (3), the control means (4), the activation means (5), the electrical power supply means (6), and the safety means.

2. An emission device according to claim 1, characterized in that the second portion of the housing (13) is a flask (14) that is hollow along its longitudinal axis (δ) to provide a cylindrical cavity (20) and that is closed at its bottom end (18) by a plug (21) serving as a base and to which it is fixed by a ring (22), and within which there is inserted a rigid cylindrical tube (25) serving as a support for the spray means (28) at the top end (19) of the flask, and as means for guiding electrical conductors (26) connecting said spray means to the electrical power supply means (6), and in that the fragrance cartridges are placed outside the flask and are secured to its top end (19).

3. An emission device according to claim 2, characterized in that each fragrance cartridge (15, 16) has an opening (30) for communicating with an inlet pipe (31) of the tube (25) allowing the fragrance to flow towards the spray means (28), and is secured to the flask (14) by engaging in a hook (32) provided outside the tube (25).

4. An emission device according to claim 1, characterized in that it includes safety means constituted by a system of electrodes associated with an electrical circuit, disposed on the housing in such a manner that when the housing is grasped in the hand, the user's hand closes said electrical circuit which delivers a signal to the electronic control means (4) of the device to cause it to switch from a sleep state to a standby state.

5. An emission device according to claim 2, characterized in that the power supply means (6) are constituted by a power electronics card (24) associated with an electric battery, both of which are placed in the bottom proton of the flask (14) and are accessible via the plug (21) for closing the flask (14).

6. An emission device according to claims 1 and 2, characterized in that the control means (4) are managed by personalized software installed in means integrated in or external to the device.

7. An emission device according to claim 1, characterized in that the control means (4) are managed by interactive software residing in a multimedia personal computer to which the device is connected.

8. An emission device according to claim 1, characterized in that the control means (4) comprise a timer for limiting the number of emissions each time a spray-triggering surface approaches the device.

9. An emission device according to claim 1, characterized in that the means (3) for spraying calibrated drops on the drops-on-demand principle comprise a plurality of ejection channels totally integrated in a silicon matrix and either of the thermal type with bubbles being created in each channel by heating so as to cause drops to appear, or of the piezoelectric type in which the ejection channels are subjected to vibrations created by piezoelectric means, thereby causing drops to be formed.

10. An emission device according to claims 1, 2, 6, 7, and 8, characterized in that the activation means (5) for activating said control means (4) are constituted by a proximity detector situated in the vicinity of the outlet zone of the spray means (3), the proximity detector activating said spray means (3) with the number of drop emissions triggered by each approach of a surface to be made fragrant to the device being limited by the timer.

11. The use of the emission device according to claim 1, characterized in that activation of the control means (4) is accompanied by visible or audible information being emitted.

12. The use of the emission device according to claim 1, characterized in that the programmable control means (4) cause a composition of fragrances to be sprayed cyclically, the composition being suitable for obtaining an odor where failure to detect the odor is an early sign of a disease.

13. The use of the emission device according to claim 1, characterized in that the programmable control means (4) cause a composition of fragrances to be sprayed, where the composition complies with a predetermined characteristic such as a season or an astral position of the user.
